# EUROPEAN PATENT SPECIFICATION

(11) **EP 3 448 342 B1**
(45) Date of publication and mention of the grant of the patent: **12.10.2022**
(21) Application number: 17723946.4
(22) Date of filing: 28.04.2017
(51) Int. Cl.: A61F 13/511, D04H 1/54, D04H 1/541, D01F 8/06, D01F 8/14, D01D 5/253, D01D 5/26, D01D 5/34

(54) **BI-COMPONENT STAPLE OR SHORT-CUT TRILOBAL FIBRES AND THEIR USES**
ZWEIKOMPONENTIGE TRILOBALFASERN UND DEREN VERWENDUNGEN
FIBRES TRILOBÉES À BICOMPOSANT ET LEURS UTILISATIONS

(30) Priority: 29.04.2016 EP 16167771; 21.10.2016 EP 16195024
(43) Date of publication of application: 06.03.2019
(73) Proprietor: Beaulieu International Group NV, 8790 Waregem (BE)
(72) Inventor: PINOCA, Leonardo, 05039 Stroncone (IT); BIAGIOTTI, Jerico, 05100 Terni (IT); BEUN, Gillis, B-8870 Izegem (BE)
(74) Representative: IPLodge bv
(86) International application number: PCT/EP2017/060246
(87) International publication number: WO 2017/186935

(56) References cited:
- EP-A1- 1 722 020
- WO-A1-2012/133753
- WO-A2-2009/006292
- US-A- 5 162 074
- US-A1- 2005 227 563

## Description

The present invention relates generally to shaped bi-component fibres and nonwovens made with these bi-component fibres and products made with the nonwovens The present invention also relates in one aspect to the improved bonding properties of a bi-component fibre with a trilobal shape, suitable for the preparation of staple and short-cut fibres and nonwoven fabrics having superior softness.

### Background

In recent years, the market of bi-component fibres, to be used as a binding agent in nonwovens, has grown significantly mainly because these systems, having a higher melting point core and a lower melting point sheath, have shown a series of advantages over mono-component "binder" fibres in several applications. However, shaped bi-component fibres are considered to increase costs so that the extra costs can outweigh any benefits.

The demand of the hygiene market for lighter and softer products is also continuously growing. In this context, nonwovens can be produced by using conventional bi-component fibres with round cross-sections and based on polyolefin polymers. For instance, a standard sheath/core bi-component fibre in which the round core is made of 50% by weight of polypropylene (with a density of about 0.91 g/cm³ and a melting temperature of around 165 °C) and a concentric sheath is made of a 50% by weight of polyethylene (with a density of about 0.95 g/cm³ and a melting temperature of around 130 °C) allows the formation of bonding spots at temperatures that lie between the melting temperatures of the two core and sheath polyolefins. Moreover, the polyethylene of the external sheath can lead to an excellent bondability of the fibres together with an excellent softness of the obtained nonwoven, whereas the polypropylene in the core can assure that the nonwoven structure maintains a suitable mechanical consistency even after a thermal treatment able to generate the bonding spots.

Bonding fibres are mentioned in several patents: US9108839, US8487026, US7959751, US7695660, US7309522, US6916752, US6911174, US4123577, US4087507 and US3322607. In particular, in US9108839 a nonwoven is disclosed comprising three separate and distinct fibres in which the thermoplastic fibres are mixed to create a random laid layer. On the other hand, the US7959751 describes a process for producing a composite material having at least one layer comprised of elements that are bonded together, the method comprising airlaying an assembly of one or more layers of randomly oriented fibrous elements. This assembly contains a thermoplastic thermally sensitive bonding fibre capable of bonding with other materials in the assembly during activation.

A nonwoven liner for a disposable diaper having improved softness, tensile strength, and moisture transfer capability is described in the US 4668566, in which at least two layers of nonwoven webs are adjacent and bonded to each other. Each nonwoven web comprises a plurality of monofilaments or fibres of a thermoplastic material. In one of the webs the monofilaments or fibres are made from polypropylene. In another of the webs the monofilaments or fibres are made from polyethylene. It is said that this nonwoven provides remarkably increased softness and tensile strength as well as other desirable properties.

Multicomponent fibres are also mentioned in the US5108820, US5336552, US5382400, in which for two component fibres or filaments, the polymers may be present in ratios of 75/25, 50/50, 25/75 or any other desired ratios.

On the other hand, to obtain properties such as opacity, barrier properties and liquids management with mono-component fibres based nonwovens, shaped fibres can be in used. For instance, as described in the US5607766 and EP0881889, sheath-core bi-component fibres comprising a core of a thermoplastic material (preferably polypropylene or polybutylene terephthalate) are completely covered with a sheath formed of polyethylene terephthalate or a copolymer. They exhibit interesting properties useful in various applications, such as ink reservoir elements for a marking or writing instrument, although the porous element may also find utility as a tobacco smoke filter. Other forms of the same product have utility in other applications where its excellent capillary, absorption and filtering properties are advantageous.

The nonwoven disclosed in the US4753934 and US4778460 comprises a plurality of fibres or monofilaments of a thermoplastic material, each monofilament having a "bilobal" cross-section. By the term "bilobal" the applicants intend to refer to a shape including an elongate substantially rectangular portion which has at each of its furthest separated ends an enlarged portion which is typically circular and which portion has a diameter greater than the thickness of the rectangle. A nonwoven web made with such bilobal shaped monofilaments results in remarkably increased softness as well as other desirable properties. WO03049589 describes a cleaning sheet that has an enhanced dirt, dust and/or debris pickup and retention characteristics. The cleaning sheet is prepared from a nonwoven web containing plurality of multicomponent multilobal filaments, wherein the multicomponent multilobal filaments have a plurality of raised lobal regions separated by depressed regions. The nonwoven web can be a single layer or a layer of a multilayer laminate and could be optionally electret treated.

All the above mentioned patents exploit the benefits of the multilobality of the fibres to their surface performance due to the increased of contact areas and improved capillarity properties.

Some nonwovens are produced by combining binder round fibre (useful to assure a suitable mechanical consistency of the web) with a shaped fibre (useful to improve the porosity and capillarity of the web) in order to exploit the main characteristics of both the systems. For instance, in the WO2012127346 a method of making a resilient tampon is described by including 70 wt% to 95 wt% absorbent fibres (such as trilobal viscose rayon fibres) and 5 wt% to 30 wt% bi-component binder fibres. However, the need of using high percentages of a standard round bi-component fibres (normally higher than a 50 wt%) to be used as binder agent to assure a suitable mechanical consistency of nonwovens (for instance made by means of air-through bonding technology) represents a limit to the use of shaped fibres in high percentage when necessary to reach some specific properties.

EP1722020A1 describes synthetic fibers having good air opening properties and useful for producing an air-laid nonwoven fabric.

### SUMMARY OF THE INVENTION

The present invention relates to a bi-component staple or short-cut fibre with a trilobal shape, and optionally crimped, according to the subject-matter of claim 1. . Such a staple or short-cut trilobal, optionally crimped, fibre can be particularly suitable for the preparation of nonwoven fabrics. An advantage of embodiments of the present invention is that nonwovens according to any of the embodiments of the present invention can have superior softness, and/or can have improved thermal-bonding properties.

One aspect of the present invention relates to a bi-component staple or short-cut, optionally crimped, fibre comprising a core and a sheath, the sheath and the core have different melting points, with the sheath melting point being lower than the core melting point, the bi-component staple or short-cut, optionally crimped, fibre having an outer trilobal shaped cross-section. The core of the bi-component staple or short-cut, optionally crimped, fibre can have a cross-section whereby the outer trilobal shaped cross-section is created by a conformal layer of sheath material applied to the core. The sheath material of a bi-component a staple or short-cut trilobal, optionally crimped, fibre is a conformal coating on the shaped core fibre. A conformal layer is a polymeric layer which 'conforms' to the contours of the shaped core. The layer preferably has a constant thickness or a substantially constant thickness. Preferably there is at least a certain amount of sheath material at each position on the surface of the core or preferably there is at least a certain amount of sheath material at a position around any circumference of the core

The core of the bi-component staple or short-cut, optionally crimped, fibre has the same or similar symmetry as the sheath, i.e. the axes of the lobes of the trilobal outer shape radiate along three angularly spaced directions, nominally with 120° between each axis. The core has a similar three point symmetry, e.g. is a delta shape, or a trilobal shape. A polymer used for the sheath of a bi-component staple or short-cut trilobal, optionally crimped, fibre is a co-polyester, a polyolefin, or an olefin copolymer and a polymer used for the core is a polyolefin such as polypropylene (PP) or an olefin copolymer such as a polypropylene copolymer or a polyester or a co-polyester. In one preferred embodiment, the polymer used for the sheath of a bi-component a staple or short-cut trilobal, optionally crimped fibre, is a polyolefin selected from polypropylene, and polyethylene (LDPE, LLDPE or HDPE). In any of the embodiments a or the polymer used for the sheath can comprise a non-grafted polyolefin component and a grafted polyolefin component, wherein the grafted polyolefin component is present in the sheath by at most 50%, preferably at most 30% and most preferably at most 10%, by weight of the sheath. The grafting monomer may be ethylenically unsaturated carboxylic acids and ethylenically unsaturated carboxylic acid anhydrides, including derivatives of such acids, and mixtures thereof, and vinyl trialkoxy silanes. Examples of the acids and anhydrides, which may be mono, di- or polycarboxylic acids, are acrylic acid, methacrylic acid, maleic acid, fumaric acid, itaconic acid, crotonic acid, itaconic anhydride, maleic anhydride and substituted maleic anhydride e.g. dimethyl maleic anhydride or citraconic anhydride, nadic anhydride, nadic methyl anhydride and tetrahydro phthalic anhydride. Examples of derivatives of the unsaturated acids are salts, imides, amides and esters e.g. mono- and disodium maleate, acrylamide, maleimide, glycidyl methacrylate and diethyl fumarate. Examples of the vinyl trialkoxy silanes are vinyl trimethoxy silane and vinyl triethoxy silane.

The sheath represents from 10-90%, can represent from 20-80%, or 30-70%, preferably from 40-60%, even more preferably from 45-55% by weight of the bi-component staple or short-cut trilobal, optionally crimped, fibre. The core represents from 90-10%, can represent from 80-20%, or 70-30%, or 60-40%, or 55-45% by weight of the bi-component staple or short-cut trilobal, optionally crimped, fibre and the sheath has the remaining amounts.

For example, there can be 45-55% by weight (of the bi-component staple or short-cut trilobal, optionally crimped, fibre) Polypropylene (PP) in the core and 55-45% by weight (of the bi-component staple or short-cut trilobal, optionally crimped, fibre) Polyethylene (PE) in the sheath.

The core can include between 10% and 90%, preferably between 20% and 80% by weight (of the bi-component staple or short-cut trilobal optionally crimped fibre) of Polypropylene (PP) and the sheath can include between 90% and 10%, preferably between 80% and 20% by weight (of the bi-component staple or short-cut trilobal, optionally crimped, fiber) of Polyethylene (PE).

The bi-component staple or short-cut trilobal, optionally crimped, fibre can have a final titre between 0.5 and 35 dtex, preferably for some applications between 0.9 and 17 dtex or 0,9 and 9 dtex.

A nonwoven structure can include the bi-component staple trilobal, optionally crimped, fibres and be produced by carded thermal bonding, carded air-through bonding, spun bond, or is melt blown. The nonwoven structure can be entangled e.g. by needle punching or hydro-entanglement.

A nonwoven structure can include bi-component short-cut trilobal fibres and can be produced by airlaying an assembly of one or more layers of randomly oriented fibres. The nonwoven structure of any of the embodiments of the present invention can have a basic weight between 10 (or 12) gsm and 170 gsm for some applications or between 100 and 1000 gsm for others.

In another embodiment an absorbent article can comprise a liquid permeable body-facing cover layer, a liquid impermeable garment-facing barrier layer and an absorbent core between the cover layer and the barrier layer, the cover layer comprising the nonwoven structure according to any of the embodiments of the invention, e.g. a nonwoven structure comprising bi-component staple or short-cut trilobal, optionally crimped, fibres.

An embodiment of the present invention also includes use of the nonwoven structure according to any of the embodiments of the invention, e.g. a nonwoven structure comprising bi-component staple or short-cut trilobal, optionally crimped, fibres as a surface sheet in absorbent articles. This use can be in absorbent articles that include beneath the surface sheet an acquisition/transportation sheet and an absorbent sheet and a liquid-impermeable backing sheet. Any of these layers may include a nonwoven structure comprising bi-component staple or short-cut trilobal, optionally crimped, fibres.

An embodiment of the invention includes the use of the nonwoven structure according to any of the embodiments of the invention in a filter, e.g. a nonwoven structure comprising bi-component staple or short-cut trilobal optionally crimped fibres.

An embodiment of the invention incudes the use of the nonwoven structure according to any of the embodiments of the invention in a carpet, rug or mat, or in upholstery e.g. a nonwoven structure comprising bi-component staple or short-cut trilobal, optionally crimped, fibres .

An embodiment of the invention incudes use of the nonwoven structure according to any of the embodiments of the invention in a dry or wet wipe.

For example, in one aspect the present invention an absorbent core, e.g. between a cover layer and a barrier layer, a surface sheet in absorbent articles, a carpet, rug or mat, upholstery, dry or wet wipe, or filters are provided comprising a nonwoven structure. The nonwoven structure comprises shaped bi-component staple or short-cut trilobal, optionally crimped, fibres bonded with themselves or with other bi-component fibres and/or with mono-componcnt fibres. The bi-component staple or short-cut trilobal, optionally crimped, fibres comprise a core and a sheath. The sheath and the core have different melting points, with the sheath melting point being lower than the core melting point. The bi-component staple or short-cut trilobal, optionally crimped, fibres have a shaped cross-section and preferably the bi-component fibres can retain their shape after a thermo-welding process. To achieve this it is preferred if the outer shape is multi-lobal then the core should have the same symmetry as the outer sheath. Hence it is preferred that if the outer shape is trilobal and the core shape is preferably trilobal, or a delta shape. Hence the sheath is a concentric layer on the core.

### BRIEF DESCRIPTION OF THE DRAWINGS

FIGURE 1 shows the as spun PP/PE bicomponent/trilobal fibres according to an embodiment of the invention.
FIGURE 2 shows the PP/PE bi-component/trilobal fibres (2,2 dtex) fibres according to an embodiment of the invention.
FIGURE 3 shows the PP/PE bi-component/round fibres (2,2 dtex).FIGURE 4 shows an optical microscope magnification of a bonding spot.
FIGURE 5 shows the typical configuration of the dynamometer during the execution of the debonding tests.
FIGURE 6 shows the debonding curves for the system PP/PE Bi-component Round + PP Mono-component Round.
FIGURE 7 shows the debonding curves for the system PP/PE Bi-component Trilobal + PP Mono-component Round.
FIGURE 8 includes TABLE A which shows the summary of the measured debonding forces in centinewtons for the analysed systems.
FIGURES 9A to 9C schematically show wipes according to embodiments of the present invention.
FIGURE 10 schematically shows a dry wipe according to an embodiment of the present invention.
FIGURE 11 shows schematically a filter according to an embodiment of the present invention.
FIGURE 12 shows schematically a carpet according to an embodiment of the present invention.

### Definitions

A "trilobal shaped fibre" has a trilobal cross-sectional geometry including three lobes defined by three tips and made of a sheath material, and a generally solid central core section running axially through the fibre. Any of the trilobal shaped fibers used in any of the embodiments of the present invention may be staple or short-cut fibers, e.g. alone, in combinations or in blends. A trilobal fibre has a lobed cross-sectional geometry including a sheath having three lobes and hence defined by three tips, and also a generally solid central core section running axially through the fibre having a similar shape to the sheath. Each outer side of the fibre (at least before thermal bonding) preferably defines a smoothly curved contour extending between each tip and a neighbouring tip, each side preferably including a concave region located at an approximate midpoint between neighbouring tips. However triangular and even convex curves can be useful for certain applications. Hence, other shapes are included within the scope of the present invention, however in any shape it is preferred if there are three four lobes presenting sheath material to the outside which has a lower melting point than the core material, e.g. 10°C difference. Preferably, each outer side of the fibre preferably defines a contour extending between each tip and a neighbouring tip, each such contour can comprise any one of the following: a straight line, a concave shape or a convex shape. In the case of the convex shape the convex shape preferably does not extend out from the core such as to extend beyond a line drawn between two adjacent tips.

**"Staple fibers"** are fibers of limited length, e.g. 20 to 120 mm or up to 300 mm. Staple fibers as used in embodiments of the present invention can be trilobal in cross-section.

**"Bi-component staple trilobal fibers"** according to any of the embodiments of the present invention can be crimped. Crimping of bi-component staple trilobal fibers can exploit, for example, molecular weight and/or stereochemistry differences of each component or can exploit a differential in the orientation level across the fiber diameter. Additionally, polymer additives like cross linkers or branching agents can also be used to create a similar effect. Fibre crimp is defined as the waviness of a fiber expressed as waves or crimps per unit length (see for example ASTM D123) or, can be defined as the diffcrcncc in distance between two points on a fiber as it lies in in an unstretched condition and the same two points when the fiber is straightened under specific tension, expressed as a percentage of the unstretched length (see for example ASTM D123).

**"Short-cut fibers"** as used with respect to any of the embodiments of the present invention are cut trilobal fibres of a length from 2 to 25 mm and are generally not crimped. Short-cut fibres as used in embodiments of the present invention can also be bi-component trilobal short-cut fibres and can be used alone or in a blend, and can be processed with wet- or air laid technologies. Crimped short-cut fibers are less preferred but can have an inherently open nature, and can be processed in air-laid applications. They can have mechanical binding properties. They can be used in wipes, filtration, or absorbent hygiene products, for example.

A **"nonwoven structure"** which can be used with the present invention may include trilobal staple optionally crimped fibres nonwovenmade by providing cut fibres of several centimetres length, e.g. 20 to 120 mm length or up to 300 mm length. These fibers can be put into bales, placed on a conveyor belt and dispersed, e.g. spread in a uniform web by a wetlaid, airlaid, or carding/crosslapping process. Nonwoven structures can be made by a wetlaid process into mats, gauzes, scrims etc. The nonwoven structure can be entangled by hydroentanglement or needle punching in any of the embodiments of the present invention.

A nonwoven structure can also be made comprising bi-component trilobal short-cut fibres of 2 to 25 mm in length, e.g. alone or in a blend. These fibers can be spread in a uniform web by an air-laid process, e.g. for making nonwoven structures for use in mats, gauzes, scrims; sheets etc. The nonwoven structure can be entangled by hydroentanglement or needle punching also in these embodiments of the present invention.

The term **"needlepunched"** means a nonwoven structure which is consolidated by passing it though one or more needleboards carrying several thousands of needles that penetrate the nonwovens repeatedly, forming a mechanically entangled structure.

### Test methods

The following test methods are to be used.

### Melting point

Melting temperatures Tmelt ("melting point") are determined according to ISO 3146, e.g. on a DSC Q2000 instrument by TA Instruments. To erase the thermal history the samples can be first heated to 200 °C and kept at 200 °C for a period of 3 minutes. The reported melting temperatures ("melting points") are then determined with heating and cooling rates of 20°C/min.
**Dimensions:** CEN/TS 14159
**Total thickness** mm: ISO 1765 whereby the tolerance is nominally ± 15 %
**Total mass per unit area** g/m² : ISO 8543 whereby the tolerance is nominally the mass ± 15 %

### Detailed description of the embodiments

### Fibre

Fibres as disclosed in this section can be used in the embodiments of the present invention of nonwovens or any embodiment of the present invention which includes a non-woven made with such fibres.

An embodiment of the present disclosure relates to manufacture of a trilobal bi-component staple or short-cut, optionally crimped, fibre. A bi-component staple or short-cut,optionally crimped, fibre comprises a core and a sheath, the sheath and the core have different melting points, with the sheath melting point being lower than the core melting point, the bi-component staple or short-cut, optionally crimped, fibre having an outer trilobal shaped cross-section. The sheath is applied as a conformal layer on the core. The bi-component staple or short-cut, optionally crimped, fibre has a triangular symmetry, with each lobe of the sheath material having an axis radiating out from the core, the three axes of the trilobal shape being spaced angularly from each other, and the core has a triangular symmetry. The core of the bi-component staple or short-cut, optionally crimped, fibre has a delta or trilobal shaped cross-section conformal with the outer trilobal shaped cross-section. The sheath is a co-polyester, a polyolefin, or a olefin copolymer and a polymer used for the core is a polyolefin, an olefin copolymer or polyester. The bi-component staple or short-cut trilobal, optionally crimped, fibre can be manufactured with 45-55% by weight of the fibre being in the core and with 55-45% by weight of the fibre being in the sheath. However other weights are included within the scope of the invention such as the core having between 10% and 90% by weight of the fibre, and the sheath has between 90% and the 10% by weight of the fibre, the core having between 20% and 80% by weight of the fibre, and the sheath has between 80% and the 20% by weight of the fibre, the core having between 30% and 70% by weight of the fibre, and the sheath has between 70% and the 30% by weight of the fibre, the core having between 40% and 60% by weight of the fibre, and the sheath has between 60% and the 40% by weight of the fibre. The bi-component staple or short-cut staple or short-cut trilobal, optionally crimped, fibre can have a final titre of between 0.5 and 35 dtex, preferably between 0.9 and 17 dtex.

In one preferred embodiment, the polymer used for the sheath of bi-component staple or short-cut trilobal, optionally crimped, fibres is a polyolefin such as polypropylene, or polyethylene (LDPE, LLDPE or HDPE) or an olefin copolymer whereas the core is a polyolefin such as polypropylene (PP) or an olefin copolymer such as a polypropylene copolymer or polyester. In accordance with any of the embodiments of the present invention the polymer used for the sheath can comprise a non-grafted polyolefin component and a grafted polyolefin component, wherein the grafted polyolefin component is present in the sheath by at most 50%, preferably at most 30% and most preferably at most 10%, by weight of the sheath. The grafting monomer may be ethylenically unsaturated carboxylic acids and ethylenically unsaturated carboxylic acid anhydrides, including derivatives of such acids, and mixtures thereof, and vinyl trialkoxy silanes. Examples of the acids and anhydrides, which may be mono, di- or polycarboxylic acids, are acrylic acid, methacrylic acid, maleic acid, fumaric acid, itaconic acid, crotonic acid, itaconic anhydride, maleic anhydride and substituted maleic anhydride e.g. dimethyl maleic anhydride or citraconic anhydride, nadic anhydride, nadic methyl anhydride and tetrahydro phthalic anhydride. Examples of derivatives of the unsaturated acids are salts, imides, amides and esters e.g. mono- and disodium maleate, acrylamide, maleimide, glycidyl methacrylate and diethyl fumarate. Examples of the vinyl trialkoxy silanes are vinyl trimethoxy silane and vinyl triethoxy silane.

The sheath can represent from 10 to 90% or 20-80%, or 30 to 70% or 40-60%, or 45-55% by weight of the bi-component staple or short-cut trilobal, optionally crimped, fibre. The core can represent the remaining amount of 90 to 10%, 80-20%, 70% to 30%, 60-40%, or 55-45% by weight of the bi-component staple or short-cut trilobal, optionally crimped, fibre, respectively.

As an example of the manufacturing method, a trilobal staple or short-cut, optionally crimped, fibre is made with a shaped polypropylene core and with a polyethylene sheath which has a lower melting temperature than the core:
Polypropylene (PP) - HC12XB (by Polychim Industrie) with a Melt Flow Rate (MFR ASTM D1238: 230 °C/2,16 Kg) of 25 g/10min, density (ASTM 1505) = 0.90 g/cm³, heat deflection temperature (ASTM D648; 455 KPa) of 105 °C, heat deflection temperature (ASTM D648; 1820 KPa) of 56 °C, Vicat softening temperature (ASTM D1525-A; 9.81 N) of 154 °C and Vicat softening temperature (ASTM D1525-A; 49.05 N) of 95 °C was spun as a trilobal core.

Polyethylene (PE) - Aspun 6834 (by Dow Chemical company), with MFR (ISO 1133: 190 °C/2.16 Kg) of 17 g/10min, density (ASTM D792) of 0.95 g/cm³ and Melting Temperature (DSC Dow method) of 130 °C was spun over the core to form a bi-component trilobal fibre. These fibres were then cut to form bi-component staple trilobal fibers in the range 20 to 120 mm or up to 300 mm or cut to form bi-component short-cut trilobal fibres of 2 to 25 mm in length. The fibres can be optionally crimped.

### Nonwoven structures

Any of the embodiments of the present invention can be a nonwoven structure comprising bi-component staple or short-cut, optionally crimped, fibres comprising a core and a sheath, the sheath and the core have different melting points, with the sheath melting point being lower than the core melting point, the bi-component fibre having an outer trilobal shaped cross-section.

Embodiments of the present invention can use through-air bonding technology in which a hot fluid (e.g. air) is forced through a preformed web. If the temperature of the fluid is high enough, the polymer of the sheath of bi-component staple or short-cut, optionally crimped, fibres can melt by forming bonds in which two or more fibres come into contact. Embodiments of the present invention can use thermal-bonding technology, in which a preformed web of fibres is passed between heated calender rolls. Bonding occurs in the areas in which the fibres are pressed by the heated rolls. On a smooth calender roll, bonding occurs wherever fibres cross each other while on an embossed calender roll, bonding occurs primarily between the raised embossed areas. This results in bonding "points" or "spots". In each of the mentioned processes, the bicomponent staple or short-cut, optionally crimped, fibres are heated and, in the zones in which the melting occurs, form a bond that is consolidated once the system is subsequently cooled. The nonwoven can be entangled by needle punching or hydro entanglement, for example.

### Wipes and hygiene products.

Any of the embodiments of the present invention can be a wipe or a hygiene product comprising a nonwoven structure made with bi-component staple or short-cut, optionally crimped, fibres comprising a core and a sheath, the sheath and the core have different melting points, with the sheath melting point being lower than the core melting point, the bi-component fibre having an outer trilobal shaped cross-section. A sheath can be a conformal layer on a core. The cores of the bi-component fibres can have a delta or trilobal shaped cross-section. The nonwoven structure can have a basic weight between 12 gsm and 170 gsm. The bi-component staple or short-cut fibre can have a triangular symmetry, with each lobe of the sheath material having axis radiating out from the core, the three axes of the trilobal shape being spaced angularly from each other, and the core has a triangular symmetry. The core of the bi-component staple or short-cut fibres can have a delta or trilobal shaped cross-section conformal with the outer trilobal shaped cross-section. A polymer used for the sheath can be a co-polyester, a polyolefin, or an olefin copolymer and a polymer used for the core is a polyolefin, an olefin copolymer or a polyester or a co-polyester. The sheath can have between 10-90% by weight of the fibre and the core can have between 90-10%% by weight of the fibre. The sheath can be made from polyethylene and can have between 45-55% by weight of the fibre and wherein the core can be made from polypropylene and can have between 55-45% by weight of the fibre. The final titre can be between 0.5 and 35 dtex, preferably between 0.9 and 17 dtex.

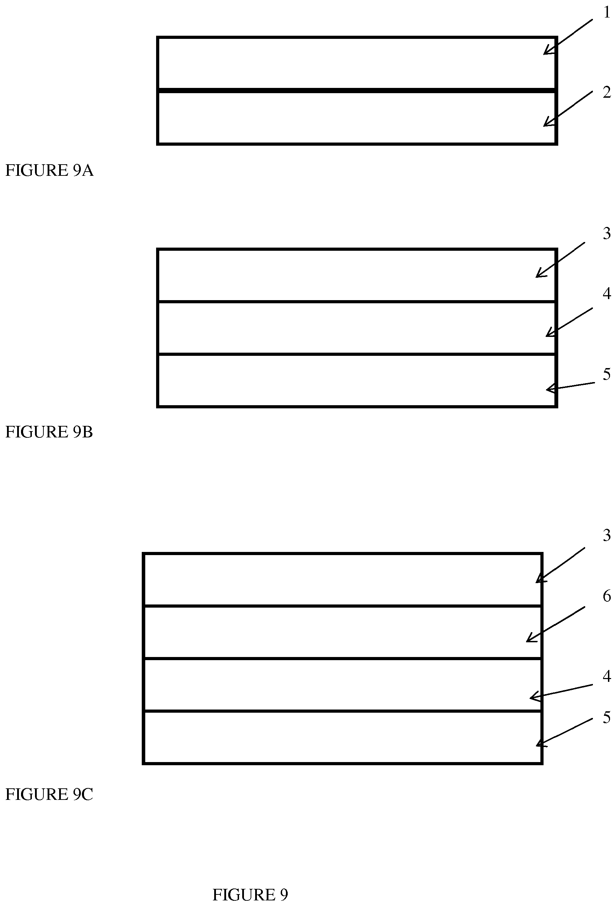

Embodiments of the present invention include gauzes, wipes, absorbent pads, hygiene products such as baby diapers, feminine liners, adult incontinence products, and the like. As shown schematically in Figure 9A these can include a top sheet (1) and/or a back sheet (2) made from or including a nonwoven structure with a weight range of 10 or 12 to 170 or from 14 to 48 gsm for example. These products can be made by calender-thermal bonding technology. For example carded veils including shaped bi-component staple, optionally crimped, fibres according to any of the embodiments of the present invention can be subjected to the action of pressure and temperature of a calender which process produces bonding spots of the bi-component staple trilobal, optionally crimped, fibre sheath material. Such products have the mechanical consistency of a nonwoven. Alternatively, such products can be made by means of air-through bonding technology using for example trilobal short-cut fibres. In this process carded veils including shaped bi-component staple or short-cut, optionally crimped, fibres according to any of the embodiments of the present invention are subjected to the action of hot-air.

Shaped bi-component staple or short-cut trilobal, optionally crimped, fibres according to any of the embodiments of the present invention can be used in the manufacture of Acquisition Distribution Layers (ADL). Typical weight ranges for ADL including shaped bi-component staple or short-cut trilobal, optionally crimped, fibres according to any of the embodiments of the present invention lie between 10 and 170 gsm as a function of the final product, e.g. baby diapers, feminine liners, adult incontinence products.

Cleaning wipes can be used for cleaning a variety of surfaces such as kitchen surfaces, upholstery, curtains, furniture surfaces, and the like.

As shown schematically in Figure 9B a wipe can comprise a first liquid permeable layer (3) and a second liquid permeable layer (4) joined possibly to a third liquid impermeable layer (5). The first layer (3) can include a nonwoven structure according to any of the embodiments of the present invention, e.g. comprising bi-component staple or short-cut trilobal, optionally crimped, fibres. A cleaning composition can be releasably absorbed into one or more of the first layer (3), the second layer (4) or a core, if present. As shown schematically in Figure 9C a core (6) can be between the first layer (3) and the second layer (4). Within the core (6), a cleaning composition can be releasably absorbed. The first liquid permeable layer can provide for transmission of cleaning composition from the core to a surface of the first layer.

Alternatively, an absorbing material such as a superabsorbent compound can held or encapsulated by one or more of the first layer (3), the second layer (4) or a core (6), if present. A core (6) can be between the first layer and the second layer. Within the core, the absorbing material can be held. The first liquid permeable layer can provide for transmission of liquids from the surface of the first layer to the core.

The first layer can be attached to the core by using any technique known in the art for joining webs of material, including, but not limited to, ultrasonic bonding, thermal bonding, thermo-welding, spray-gluing, for example. Alternatively, the core can be encapsulated in a pocket formed by the first layer and the second layer without the core being attached to the first layer and second layer.

As the first layer includes a nonwoven structure according to any of the embodiments of the present invention comprising bi-component trilobal staple or short-cut, optionally crimped, fibres it possesses a soft feeling and is mechanically strong. The first layer may be a composite or laminate made of a nonwoven structure according to any of the embodiments of the present invention another layer selected from thermoplastic films, porous films, reticulated foams, natural fibres especially cotton fibres.

The first layer can be hydrophobic, however the outer and/or inner surfaces of the first layer can be made hydrophilic by treatment with a surfactant which is substantially evenly and completely distributed throughout the surface of the first layer.

The core can be a material that can releasably absorb a cleaning composition or can hold an absorbing material such as a superabsorbent material. In practice, the voids within the core can act as a reservoir for the cleaning composition or the absorbing material, the cleaning composition or the absorbing material being stored within the capillaries within the core. The core can be a fibrous material in which the capillaries are provided by the interstitial spaces between the fibres of the core. The core can be an open-celled foam in which the capillaries are provided by the interconnected pores within the foam. An economical core can be provided by a nonwoven comprising polyolefin fibres according to any of the embodiments of the present invention.

The second layer can be liquid permeable. That is, the second layer can also provide for transmission of liquid cleaning composition from a core to a surface of the second layer or from the surface to the core.

The second layer can be made of a nonwoven comprising polyolefin fibres according to any of the embodiments of the present invention comprising bi-component trilobal staple or short-cut, optionally crimped, fibres and hence is compliant and has a soft feeling.

The wipe can have an abrasive layer. The abrasive layer of the wipe can be the second layer of the wipe. In such a wipe the first layer comprises a nonwoven structure according to any of the embodiments of the present invention comprising bi-component trilobal staple or short-cut, optionally crimped, fibres and provides a soft and strong wiping surface and the abrasive layer can be on the side of the core opposite to the first layer. For example, the wipe can have 3 layers, a first layer, an abrasive layer being the second layer, and a core disposed between the abrasive layer and first layer.

The second layer can be located between the abrasive layer and the core. A suitable abrasive layer can be manufactured from a wide range of materials such as thermoplastic films, porous plastic films, reticulated foams, natural fibres of which cotton fibres are preferred, or a nonwoven structure according to any of the embodiments of the present invention comprising bi-component trilobal staple or short-cut, optionally crimped fibres.

The use of the shaped bi-component trilobal staple or short-cut, optionally crimped, fibres according to any of the embodiments of the present invention provides improved bondability and can be exploited in top sheets, back sheets and ADL systems, to allow the use of lower amounts of the shaped bi-component trilobal staple or short-cut, optionally crimped, fibres in blends with other fibres by maintaining the same mechanical consistency of the final nonwovens, to reduce the base weight of the nonwoven and by maintaining good thickness levels and excellent coverage effects. Moreover, the use of the shaped bi-component trilobal staple or short-cut, optionally crimped, fibres in the nonwovens for top sheets and back sheets can improve the bonding with the polymer film (e.g. polyethylene) with which these systems arc often couplcd.

Wipes including shaped bi-component staple or short-cut, optionally crimped, fibres according to any of the embodiments of the present invention can have weights in the range 10 (or 12) to 170, or 27 to 45 gsm. They can be manufactured by using the calender-thermal bonding process or for example spunlace technology. In the spunlacing process the mechanical consistency of the produced nonwovens including shaped bi-component staple or short-cut, optionally crimped, fibres according to any of the embodiments of the present invention is reached by the mechanical bonding generated by hydro-entanglement, in which high-speed jets of water strike the web so that the fibres knot about one another. Alternatively needle punching can be used. A thermal-calendering process can be used after the hydro-entanglement or needle punching in order to fix better the mechanical bonding and to improve the mechanical characteristics of the nonwovens. The use of the shaped bi-component staple or short-cut trilobal, optionally crimped, fibre according to any of the embodiments of the present invention in wipes can improve the calendering process, and improved bulkiness, liquid behaviour and also the stability that can be realized by using lower temperatures.

For what concerns the shape of the shaped bi-component staple or short-cut, optionally crimped, fibres according to any of the embodiments of the present invention, the sheath preferably has a trilobal shape and the core has a shape which has a symmetry similar to that of the sheath, e.g. a delta shape or trilobal. Preferably both the core and the sheath have a trilobal shape.

### Dry wipes

Any of the embodiments of the present invention can be a dry wipe or a hygiene product comprising a nonwoven structure made with bi-component staple or short-cut, optionally crimped, fibres comprising a core and a sheath, the sheath and the core have different melting points, with the sheath melting point being lower than the core melting point, the bi-component fibre having an outer trilobal shaped cross-section. A sheath can be a conformal layer on a core. The cores of the bi-component fibres can have a delta or trilobal shaped cross-section. The nonwoven structure has a basic weight between 12 gsm and 170 gsm. The bi-component staple or short-cut fibre can have a triangular symmetry, with each lobe of the sheath material having axis radiating out from the corc, the three axes of the trilobal shape being spaced angularly from each other, and the core has a triangular symmetry. The core of the bi-component staple or short-cut fibres has a delta or trilobal shaped cross-section conformal with the outer trilobal shaped cross-section. A polymer used for the sheath can be a co-polyester, a polyolefin, or an olefin copolymer and a polymer used for the core is a polyolefin, an olefin copolymer or a polyester or a co-polyester. The sheath can have between 10-90% by weight of the fibre and the core can have between 90-10%% by weight of the fibre. The sheath can be made from polyethylene and can have between 45-55% by weight of the fibre and wherein the core can be made from polypropylene and can have between 55-45% by weight of the fibre. The final titre can be between 0.5 and 35 dtex, preferably between 0.9 and 17 dtex.

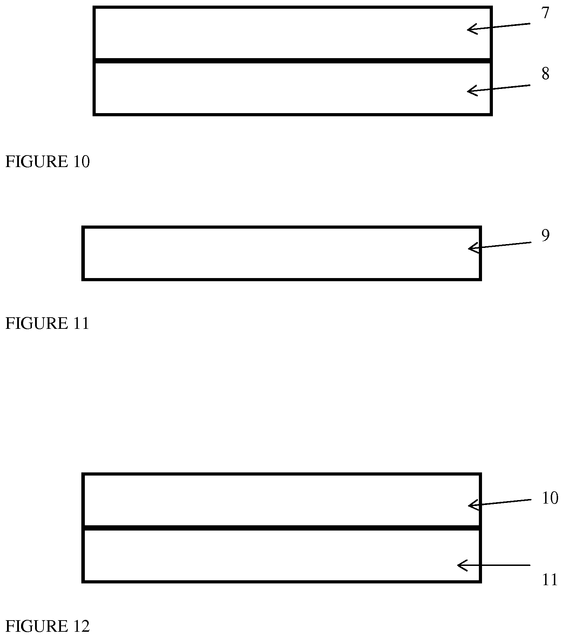

As shown schematically in Figure 10 a dry wipe according to an embodiment of the present invention comprises a nonwoven structure (7) according to any of the embodiments of the present invention having trilobal bi-component staple or short-cut, optionally crimped, fibres. Dry wipes according to embodiments of the present invention include shaped bi-component fibres according to any of the embodiments of the present invention which have been bonded to themselves or to mono-component fibres. The shaped bi-component staple or short-cut optionally crimped fibres permit good inter-fibre thermal bonding (e.g., in thru-air dryers or bonding ovens, through infra-red (IR) or radiofrequency (RF) heating, etc.) and are preferably trilobal in outer sheath shape. The shaped bi-component fibres provide softness.

The wipe may also include in one or more layers (8) of natural fibres, synthetic fibres, or mixtures of natural and synthetic fibres. Natural fibres may include cellulosic fibres, such as wood pulp fibres, cotton, and rayon. Synthetic fibres may include fibres such as, polyolefins, for example polyester and polypropylene fibres. The wipe may be capable of being flushed in a toilet (i.e., it is "flushable"). For example, the thermal bonding treatment may be made in discrete zones so that the wipe may disintegrate into pieces sufficiently small, such that when being transported in the sewer system the pieces do not plug any element of the sewer system.

The wipe can be any size or shape that may be used for cleansing the skin, or providing other benefits when using or changing a hygiene article. For example, in certain embodiments, the wipe may be rectangular or circular. In certain embodiments, the wipe may be about 25 square centimeters in size to about 50 square centimeters in size. In certain other embodiments, the wipe may be between about 100 square centimeters in size to about 320 square centimeters) in size.

The wipe may be textured, patterned embossed, dyed, printed with ink, clear polymer or colored polymer, or combinations thereof. For example, the wipe may be printed or dyed to give a visual signal of an active ingredient. The wipe may be patterned by hydroforming or any other method known in the art.

Dry wipes including shaped bi-component staple or short-cut, optionally crimped, fibres according to any of the embodiments of the present invention can have weights in the range 10 or 12 to 170 or 27-45 gsm. They can be manufactured by using the calender-thermal bonding process or for example spunlace technology. In the spunlacing process the mechanical consistency of the produced nonwovens including shaped bi-component staple or short-cut, optionally crimped, fibres according to any of the embodiments of the present invention is reached by the mechanical bonding generated by hydro-entanglement, in which high-speed jets of water strike the web so that the fibres knot about one another. Alternatively needle punching can be used. A thermal-calendering process can be used after the hydro-entanglement or needle punching in order to fix better the mechanical bonding and to improve the mechanical characteristics of the nonwovens. The use of the shaped bi-component trilobal staple or short-cut, optionally crimped, fibre according to any of the embodiments of the present invention in wipes can improve the calendering process, and improved bulkiness, liquid behaviour and also the stability that can be realized by using lower temperatures.

### Filters

Any of the embodiments of the present invention can be a filter comprising a nonwoven structure made with bi-component staple or short-cut, optionally crimped, fibres comprising a core and a sheath, the sheath and the core have different melting points, with the sheath melting point being lower than the core melting point, the bi-component fibre having an outer trilobal shaped cross-scction. A sheath can be a conformal layer on a core. The cores of the bi-component fibres can have a delta or trilobal shaped cross-section. The nonwoven structure has a basic weight between 12 gsm and 170 gsm. The bi-component staple or short-cut fibre can have a triangular symmetry, with each lobe of the sheath material having axis radiating out from the core, the three axes of the trilobal shape being spaced angularly from each other, and the core has a triangular symmetry. The core of the bi-component staple or short-cut fibres has a delta or trilobal shaped cross-section conformal with the outer trilobal shaped cross-section. A polymer used for the sheath can be a co-polyester, a polyolefin, or an olefin copolymer and a polymer used for the core is a polyolefin, an olefin copolymer or a polyester or a co-polyester. The sheath can have between 10-90% by weight of the fibre and the core can have between 90-10%% by weight of the fibre. The sheath can be made from polyethylene and can have between 45-55% by weight of the fibre and wherein the core can be made from polypropylene and can have between 55-45% by weight of the fibre. The final titre can be between 0.5 and 35 dtex, preferably between 0.9 and 17 dtex.

As shown schematically in Figure 11a filter can include a nonwoven layer (9) including shaped bi-component staple or short-cut trilobal, optionally crimped, fibres according to any of the embodiments of the present invention, have the properties of base weight, porosity, fibre denier, and other factors. These factors affect filter performance, such as filtration efficiency, dust-holding capacity, air permeability, etc. Typically, there is a trade-off when designing these filters. With an increase in filter efficiency there is usually a decrease in air permeability, an increase in base weight, or some combination of both.

Filters including trilobal bi-component staple or short-cut, optionally crimped, fibres according to any of the embodiments of the present invention can increase efficiency without increasing base weight or sacrificing permeability. Furthermore, nonwovens including bi-component staple or short-cut trilobal, optionally crimped, fibres according to any of the embodiments of the present invention can have improved stiffness. The improvements can be realised in comparison with round fibres.

Embodiments of the filters include shaped bi-component staple or short-cut trilobal, optionally crimped, fibres according to any of the embodiments of the present invention which have been bonded to themselves or to mono-componcnt fibres. The shaped bi-component staple or short-cut, optionally crimped, fibres permit good inter-fibre thermal bonding (e.g., in thru-air dryers or bonding ovens, through infra-red (IR) or radio frequency (RF) heating, etc.) and are preferably trilobal in outer sheath shape. The shaped bi-component staple or short-cut trilobal, optionally crimped, fibres increase filter efficiency without significantly adversely affecting permeability, as compared to nonwovens with round fibres and equivalent base weights.

Nonwovens of shaped bi-component fibres including bi-component staple or short-cut trilobal, optionally crimped, fibres according to any of the embodiments of the present invention either alone or mixed with mono-component fibres can achieve higher filter efficiencies, yet have substantially the same equivalent base weight and tensile strength as nonwovens made of round fibres. The nonwovens can be made by a dry laid processing or thru-air bonding applications. Shaped bi-component staple or short-cut trilobal, optionally crimped, fibres according to any of the embodiments of the present invention can be thermoplastic staple or short-cut, optionally crimped, fibres having a linear mass density of between approximately 0.5 dtex and about 30 dtex. In some embodiments, mono-component fibres can be included and can be also thermoplastic staple or short-cut, optionally crimped, fibres having a linear mass density of between about 0.5 dtex and approximately 30 dtex. In various different embodiments, the shaped bi-component staple or short-cut, optionally crimped, fibres according to any of the embodiments of the present invention have an outer cross-sectional shape that is trilobal, with the core being delta shaped, or trilobal.

For some embodiments, the shaped bi-component staple or short-cut trilobal, optionally crimped, fibres according to any of the embodiments of the present invention comprise a core and a sheath, with the core having a higher melting point than the sheath. Thus, when heated, the sheath can become molten before the core. This permits the sheath of the shaped bi-component fibres to function as a bonding material, whereby the core and the sheath should maintain structural integrity of the shaped fibres. Due to surface tension, a molten sheath may attempt to circularise itself, i.e. to reduce its surface energy. By the core being of a similar shape to the sheath the outer shape can be maintained after melting. After bonding the core and sheath of the shaped bi-component staple or short-cut trilobal, optionally crimped, fibres a network structure is provided with good tensile strength, stiffness, and porosity of the nonwoven. Preferably, the shaped bi-component staple or short-cut trilobal, optionally crimped, fibres have a linear mass density of between approximately 0.5 dtex and approximately 30 dtex.

The shaped cross-section of the bi-component staple or short-cut, optionally crimped, fibres according to any of the embodiments of the present invention increases the available surface area of these fibres during filtration, thereby increasing the interface where the bi-component staple or short-cut, optionally crimped, fibres can interact with diffusing particles during filtration. For example, a trilobal cross-sectional shape of the bi-component staple or short-cut, optionally crimped, fibres increases the tortuous length of the diffusion path, thus increasing filtration efficiency without increasing base weight.

If addition, mono-component fibres which can be included in the nonwoven, need not be thermoplastic, since the mono-component fibres are not the main bonding fibres. Thus, the mono-component fibres can be acrylic, glass, or other non-thermoplastic fibres. However, thermoplastic mono-component fibres may have advantages, such as, for example, better bonding affinity to the shaped bi-component fibres. For some embodiments, polypropylene shaped mono-component fibres can be used because polypropylene is the lowest density polymer for a given mass linear density (e.g. for a given dtex), thereby providing greater surface area for a given dtex, as compared to other polymers. The lower density, therefore, results in greater filtration ability to filter, better bonding characteristics, better ability to charge medium, and advantageous triboelectric effects.

For some embodiments, it should be noted that in addition to shaped bi-component staple or short-cut trilobal, optionally crimped, fibres according to any of the embodiments of the present invention, shaped mono-component fibres can be used in conjunction with the shaped bi-component staple or short-cut, optionally crimped, fibres to increase the surface area. For other embodiments, one can appreciate that shaped bi-component fibres can also be used to further increase surface area. It should also be noted that a polypropylene sheath with a higher-melting-temperature polyester core can be used.

### Carpets, Mats, Upholstery

Any of the embodiments of the present invention can be carpet, mat or rug, upholstery comprising a nonwoven structure made with bi-component staple or short-cut, optionally crimped, fibres comprising a core and a sheath, the sheath and the core have different melting points, with the sheath melting point being lower than the core melting point, the bi-component fibre having an outer trilobal shaped cross-section. A sheath can be a conformal layer on a core. The cores of the bi-component fibres can have a delta or trilobal shaped cross-section. The nonwoven structure has a basic weight between 12 gsm and 170 gsm. The bi-component staple or short-cut fibre can have a triangular symmetry, with each lobe of the sheath material having axis radiating out from the core, the three axes of the trilobal shape being spaced angularly from each other, and the core has a triangular symmetry. The core of the bi-component staple or short-cut fibres has a delta or trilobal shaped cross-section conformal with the outer trilobal shaped cross-section. A polymer used for the sheath can be a co-polyester, a polyolefin, or an olefin copolymer and a polymer used for the core is a polyolefin, an olefin copolymer or a polyester or a co-polyester. The sheath can have between 10-90% by weight of the fibre and the core can have between 90-10%% by weight of the fibre. The sheath can be made from polyethylene and can have between 45-55% by weight of the fibre and wherein the core can be made from polypropylene and can have between 55-45% by weight of the fibre. The final titre can be between 0.5 and 35 dtex, preferably between 0.9 and 17 dtex.

As shown schematically in Figure 12 a nonwoven structure (10) according to embodiments of the present invention can be used in carpets, rugs, mates and textile floor and wall covering in general. The nonwoven structure may have a needlefelt structure. An advantage of event or exhibition carpet according to embodiments of the present invention is a reduction weight and/or cost, while keeping or improving performance such as good abrasion resistance.

In accordance with embodiments of the present invention a floor covering such as carpet rug or mat may include a facing layer including a nonwoven structure according to any of the embodiments of the present invention comprising bi-component staple or short-cut trilobal, optionally crimped, fibres. The floor covering may include at least 50% by weight of trilobal staple or short-cut bi-component, optionally crimped, fibres, and at least a partial thermal bonding of the fibres.

The trilobal bi-component staple or short-cut, optionally crimped, fibre content of the facing layer can be at least 60%, at least 70%, at least 80% or at least 90% by weight of the total fibre content in the facing layer, up to 100% by weight.

The bi-component trilobal staple or short-cut, optionally crimped, fibres of the facing layer are (at least partially) bound by a thermal bonding process. Further bonding methods may be applied in addition, e.g. by latex, or bonding powder. A backing layer (11) can be applied but is less preferred especially if it has a significant impact one the overall manufacturing cost

Carpets, rugs or mats, upholstery, or floor or wall covering textiles in general can include shaped bi-component staple or short-cut trilobal, optionally crimped, fibres according to any of the embodiments of the present invention can have weights in the range 100 to 1000 gsm, typically in the range 200 to 600 gsm or 150 to 350 gsm. In a preferred embodiment the weight of the nonwoven structure used as a top or facing layer (base weight) is between 100 and 350 grams per square meter, for example more preferred between 150-275 grams per square meter. Fibre linear mass densities are preferably between 3.3 until 17 dtex, whereby there can be a mixture of linear mass densities of the fibre within one carpet. For example flat and structured carpet can be made with a fibre of 8.9 dtex, white flat and structured carpet can have a mixture of 3.3, 6.7 and 8.9 dtex. Fibres up to 17 dtex can be used for event carpet with velour qualities, e.g. from 7 to 17 or from 9 to 17 dtex.

Carpets according to embodiments of the present invention can be manufactured by using the calender-thermal bonding process or for example spunlace technology or needle punching. In the spunlacing process the mechanical consistency of the produced nonwovens including shaped bi-component staple or short-cut, optionally crimped, fibres according to any of the embodiments of the present invention is reached by the mechanical bonding generated by hydro-entanglement, in which high-speed jets of water strike the web so that the fibres knot about one another. However, a thermal-calendering process can be used after the hydro-entanglement in order to fix better the mechanical bonding and to improve the mechanical characteristics of the nonwovens or needle punching can be used. The use of the shaped bi-component staple or short-cut trilobal, optionally crimped, fibre according to any of the embodiments of the present invention in textiles can improve the calendering process, and improved bulkiness, liquid behaviour and also the stability that can be realized by using lower temperatures.

Carpets according to embodiments of the present invention have good coverage while having a low weight. Good coverage could be provided by a high weight dense fibre packing as this places as much polymer material as possible to block transmitted light in any cross-section of the carpet. So a compact fibre density would provide good coverage but would increase weight. In accordance with embodiments of the present invention the lobed nature of the fibre creates "lobe tip-to-adjacent fibre" and "lobe-to-lobe" touching which spaces the fibres from each other. This form of packing allows a low weight with a high coverage in which air replaces the polymer as much as possible. The tips of the lobes preferably have convex surfaces.

The facing layer can be printed, e.g. preferably digitally printed so that the carpet can be customized to a requirement rather than stocking large quantities of pre-customised carpet.

### Comparative Test samples

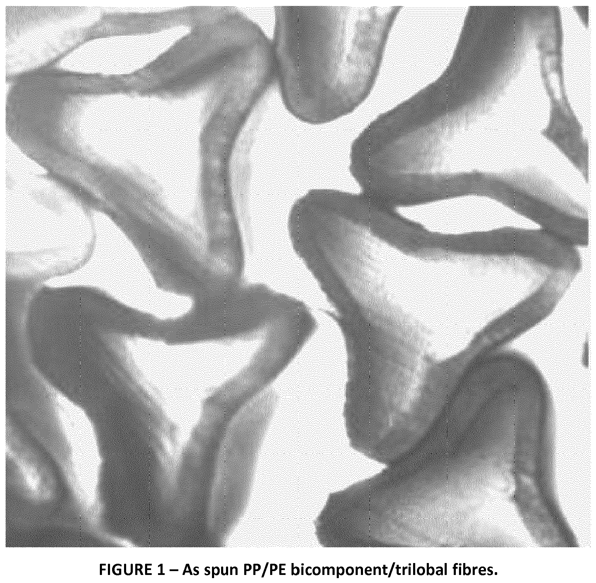

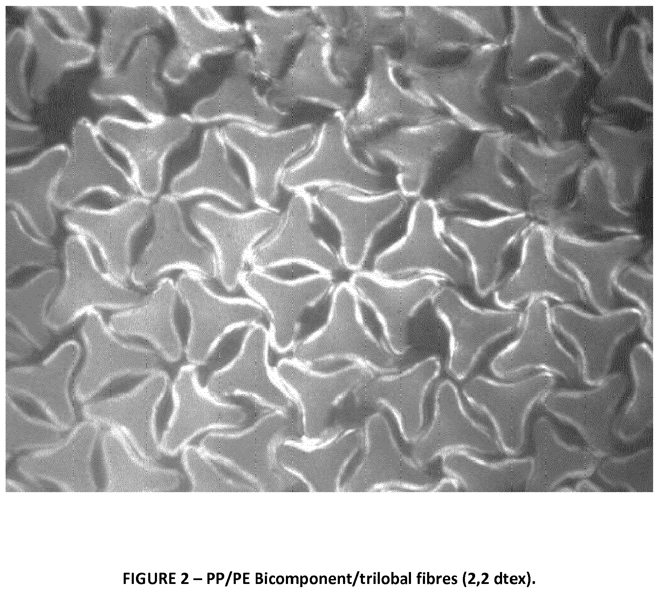

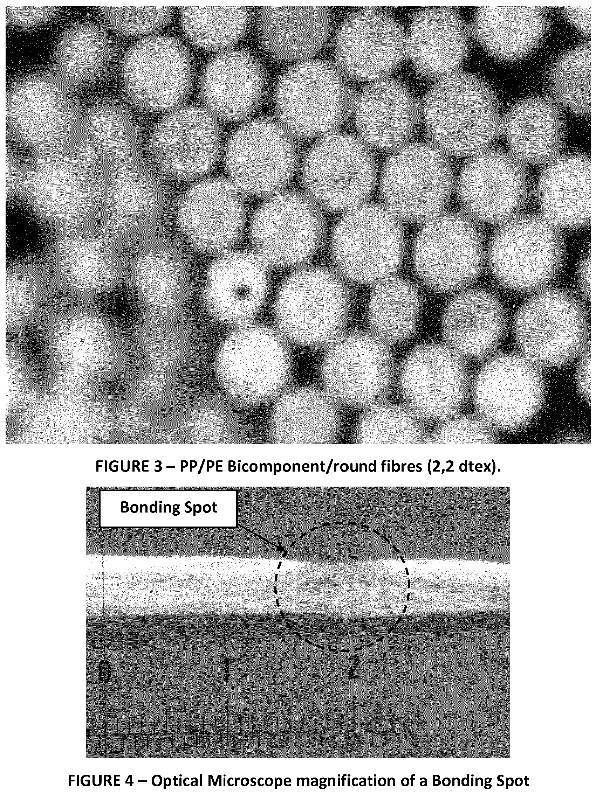

Bundles of 36 filaments, bi-component (50 wt% PP/50 wt% PE) and mono-component (100 wt% PP), having a trilobal (as illustrated in Figure 1 and 2) and round sections (as illustrated in Figure 3), needed for the following described evaluations, have been produced by using a spinning line. All these fibres have been obtained by maintaining the same raw materials, the same core/sheath ratio (50% core/50% sheath), the same process conditions and the same final titre of 2,2 dtex (the dtex is defined as the weight, expressed in grams, of 10.000 m of fibre).

The polymers used to produce these filaments were the Polypropylene Homopolymer (PP) HC12XB (by POLYCHIM INDUSTRIE) and the Polyethylene (PE) Aspun 6834 (by DOW CHEMICAL COMPANY), having thermal and physical characteristics as following detailed:

### Polypropylene (PP) - HC12XB (by POLYCHIM INDUSTRIE):

▪ *MFR (ASTM D1238: 230 °C*/*2,16 Kg)* = *25 g*/*10min*
▪ *Density (ASTM 1505)* = *0.90 g*/*cm³*
▪ *Heat deflection temperature (ASTM D648; 455 KPa)* = *105 °C*
▪ *Heat deflection temperature (ASTM D648; 1820 KPa)* = *56 °C*
▪ *Vicat softening temperature (ASTM D1525-A; 9.81 N)* = *154 °C*
▪ *Vicat softening temperature (ASTM D1525-A; 49.05 N)* = *95 °C*

### Polyethylene (PE) - Aspun 6834 (by DOW CHEMICAL COMPANY):

▪ *MFR (ISO 1133: 190 °C*/*2.16 Kg)* = *17 g*/*10min*
▪ *Density (ASTM D792)* = *0.95 g*/*cm³*
▪ *Melting Temperature (DSC Dow method)* = *130 °C*

The produced fibres bundles systems are following listed together with the adopted production settings:

### PP/PE BICOMPONENT TRILOBAL FIBRES (BT)

*Extruder A: Polypropylene (PP)*
*Zone 1 = 195°C* /*Zone 2 = 215°C* /*Zone 3 = 230°C*
*Distribution pipeline = 235°C* /*Spin-head*= *230-235°C*
*Extruder B: Polyethylene (PE)*
*Temperature profiles: Zone 1 = 195°C* / *Zone 2 = 215°C* / *Zone 3 = 230°C*
*Distribution pipeline = 235°C* /*Spin-head*= *230-235°C*
*Total Throughput = 0.4 g*/*h*/*min*
*Ratio Core [PP] = 50 %*
*Ratio Sheath [PE] = 50 %*
*Speed Denier Roll = 1800 m*/*min*
*Speed Relax Roll = 1800 m*/*min*
*Titre = 2.2 dtex*
*Fibre shape = Trilobal*

### PP/PE BICOMPONENT ROUND FIBRES (BR)

*Extruder A: Polypropylene (PP)*
*Zone 1 = 195°C* /*Zone 2 = 215°C* /*Zone 3 = 230°C*
*Distribution pipeline = 235°C* /*Spin-head*= *230-235°C*
*Extruder B: Polyethylene (PE)*
*Temperature profiles: Zone 1 = 195°C* /*Zone 2 = 215°C* /*Zone 3* = *230°C*
*Distribution pipeline = 235°C* /*Spin-head*= *230-235°C*
*Total Throughput = 0.4 g*/*h*/*min*
*Ratio Core [PP] = 50 %*
*Ratio Sheath [PE] = 50 %*
*Speed Denier Roll = 1800 m*/*min*
*Speed Relax Roll = 1800 m*/*min*
*Titre = 2.2 dtex*
*Fibre shape = Round*

### PP MONOCOMPONENT TRILOBAL FIBRES (MT)

*Extruder A: Polypropylene (PP)*
*Zone 1 = 195°C* /*Zone 2 = 215°C* /*Zone 3 = 230°C*
*Distribution pipeline = 235°C* /*Spin-head*= *230-235°C*
*Extruder B: Polypropylene (PP)*
*Temperature profiles: Zone 1 = 195°C* / *Zone 2 = 215°C* / *Zone 3* = *230°C*
*Distribution pipeline = 235°C* /*Spin-head*= *230-235°C*
*Total Throughput = 0.4 g*/*h*/*min*
*Ratio Core [PP] = 50 %*
*Ratio Sheath [PE] = 50 %*
*Speed Denier Roll = 1800 m*/*min*
*Speed Relax Roll = 1800 m*/*min*
*Titre = 2.2 dtex*
*Fibre shape = Trilobal*

### PP MONOCOMPONENT ROUND FIBRES (MR)

*Extruder A: Polypropylene (PP)*
*Zone 1 = 195°C* /*Zone 2 = 215°C* /*Zone 3 = 230°C*
*Distribution pipeline = 235°C* /*Spin-head*= *230-235°C*
*Extruder B: Polypropylene (PP)*
*Temperature profiles: Zone 1 = 195°C* / *Zone 2 = 215°C* / *Zone 3* = *230°C*
*Distribution pipeline = 235°C* /*Spin-head*= *230-235°C*
*Total Throughput = 0.4 g*/*h*/*min*
*Ratio Core [PP] = 50 %*
*Ratio Sheath [PE] = 50 %*
*Speed Denier Roll = 1800 m*/*min*
*Speed Relax Roll = 1800 m*/*min*
*Titre = 2.2 dtex*
*Fibre shape = Round*

### Evaluation of the bonding properties of the fibres

For the evaluation of the thermal-bondability of fibres, a nonwoven fabric is prepared by using the fibres to be tested by calendering the web under controlled conditions. Subsequently, the tensions needed to tear apart the nonwoven fabric, both in the direction parallel to and transverse to the calendering direction, are measured. The tension values determined in this way are an indirect measure of the thermo-welding capability of the fibres.

The numerical results, however, can be substantially influenced by the finishing characteristics of the fibres such as crimping, surface finishing, thermosetting, etc., and by the homogeneity of distribution of the web entering the calender. In order to avoid these inconveniences and obtain a direct evaluation of the thermo-bondability characteristics of the fibres a method has been perfected as described in the EP0629720 and EP0391438.

The tested specimens have been prepared by coupling 2 of the above described uncrimped fibre bundles (36 filament for each) above described, made up of fibres 30 cm long, by forming a unique bundle of 72 filaments (around 158.4 dtex). The roving has been then twisted sixty times by means of a twist measuring device (Carderara Bossi S.p.A.) and the two extremities are united, thus obtaining a product where the two halves of the roving are entwined as in a rope. The bonding spots are then carried out on the above described specimen by using a Bruggel HSG-ETK thermo-welding equipment, operating at a plate temperature of 150°C, and by using a clamping pressure of 300 N and 0.5 seconds welding times. A typical obtained bonding spot is illustrated in Figure 4.

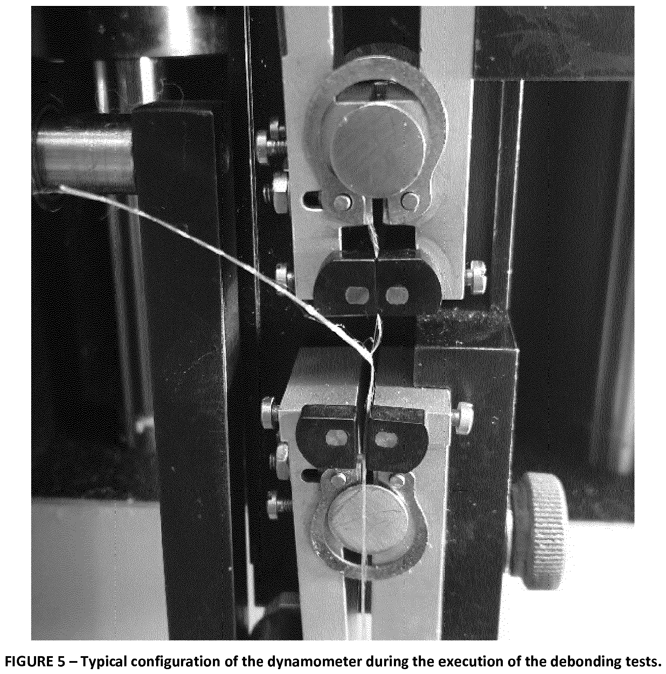

A dynamometer (Vibrodyn - Lenzing AG) is used to measure the average force required to separate the two halves of the roving which constitute each specimen at the thermo-bonded point. The distance between the clamps of the dynamometer was of 10 mm and the cross head was set at a speed of 20 mm/min by using the same configuration of the instrument illustrate in Figure 5.

The results, expressed in centinewton (cN), were obtained by averaging out at least ten measurements, and represent the thermal-bonding capacity of the tested systems.

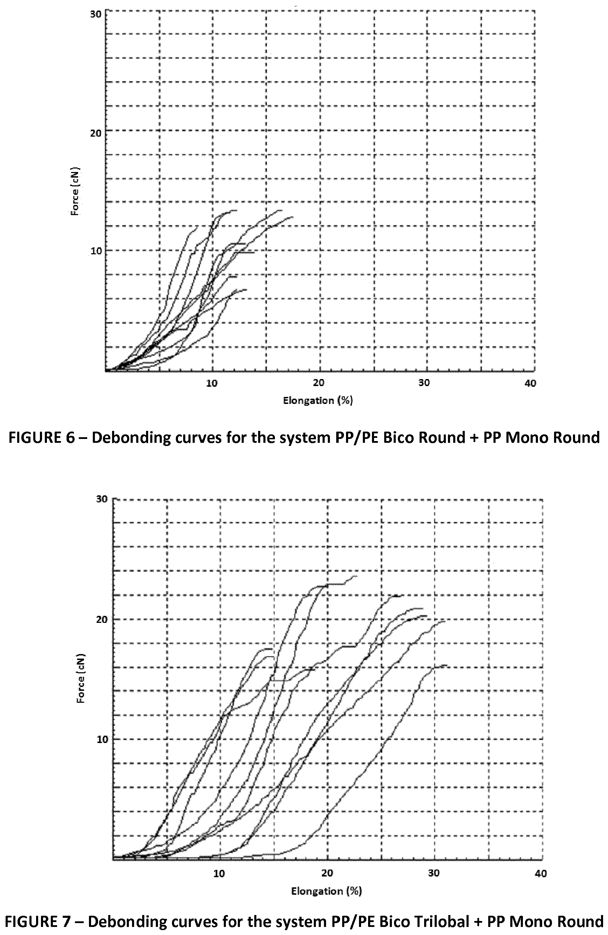

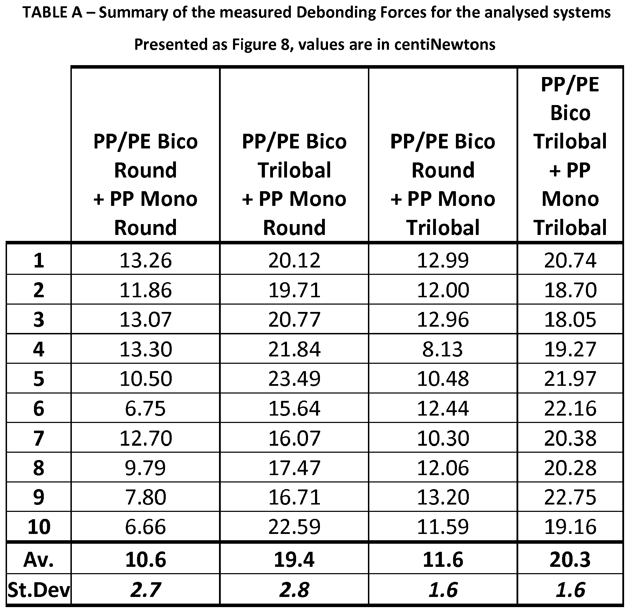

During the execution of the tests, has surprisingly been observed how the samples containing PP/PE Bi-component trilobal fibres exhibited higher debonding forces (Figures 7) in comparison with the systems in which the bi-component filaments have a round cross-section shape (Figure 6). These obtained results, summarized in the Table A (figure 8), are proving an unexpected improvement of the bondability properties of the bi-component fibres when their cross-section is trilobal. The PP/PE bi-component Trilobal fibres, with their proven improved thermal-bondability, can be used for the production of nonwovens having a higher mechanical strength, for reducing the nonwovens weights by maintaining a good coverage levels or to increase the average thickness of the nonwovens by maintaining equal weight.

## Claims

1. A bi-component staple or short-cut fibre comprising a core and a sheath, the sheath and the core having different melting points, with the sheath melting point being lower than the core melting point, the bi-component fibre having an outer trilobal shaped cross-section, wherein the fibre has a triangular symmetry, with each lobe of the sheath material having axes radiating out from the core, the three axes of the trilobal shape being spaced angularly from each other, and wherein the core has a triangular symmetry, the sheath being between 10-90% by weight of the fibre and the core being between 90-10% by weight of the fibre and wherein the sheath is made of a co-polyester, a polyolefin, or an olefin copolymer, and a polymer used for the core is a polyolefin, or an olefin copolymer, or a polyester or a co-polyester, and the core has a trilobal or a delta shaped cross-section conformal with the outer trilobal shaped cross-section, and the sheath is a conformal layer on the core.

2. The bi-component staple or short-cut fibre of claim 1, wherein the sheath is made of polyethylene (PE) and the core is made of polypropylene (PP).

3. The bi-component staple or short-cut fibre of claim 1 or claim 2, wherein the sheath is made from polyethylene and has between 30-70% by weight of the fibre and wherein the core is made from polypropylene and has between 70-30% by weight of the fibre.

4. The bi-component staple or short-cut fibre of any previous claim, in which the final titre is between 0.5 and 35 dtex, preferably between 0.9 and 17 dtex.

5. A nonwoven structure having an entanglement of staple or short-cut fibres, the staple or short-cut fibres comprising bi-component fibres thermally bonded to bi-component fibres, the bi-component fibres being according to any previous claim.

6. A non-woven structure according to claim 5, wherein the sheath is made of polyethylene (PE) and the core is made of polypropylene (PP).

7. A nonwoven structure of any of claims 5 or 6, wherein the nonwoven structure has a basic weight between 12 gsm and 170 gsm.

8. A nonwoven structure comprising shaped bi-component staple or short-cut trilobal fibres according to any of claims 1 to 4, which fibres are bonded with themselves or with other bi-component fibres and/or with mono-component fibres.

9. Use of the nonwoven structure according to any of claims 5 to 8 as a surface sheet in absorbent articles or use in an absorbent article comprising a liquid permeable body-facing cover layer, a liquid impermeable garment-facing barrier layer and an absorbent core between the cover layer and the barrier layer, the cover layer comprising the nonwoven structure according to any of claims 5 to 8.

10. Use according to claim 9, in absorbent articles that include beneath the surface sheet an acquisition/transportation sheet and an absorbent sheet and a liquid-impermeable backing sheet.

11. Use of the nonwoven structure according to any of claims 5 to 8, in a filter in a hygiene product, in a carpet, rug or mat, upholstery, or in a dry or wet wipe.

## Patentansprüche

1. Bikomponenten-Stapelfaser oder -Kurzschnittfaser, umfassend einen Kern und einen Mantel, wobei der Mantel und der Kern unterschiedliche Schmelzpunkte aufweisen, wobei der Mantelschmelzpunkt niedriger ist als der Kernschmelzpunkt, wobei die Bikomponentenfaser einen äußeren trilobal geformten Querschnitt aufweist, wobei die Faser eine dreieckige Symmetrie aufweist, wobei jeder Lappen des Mantelmaterials Achsen aufweist, die vom Kern strahlenförmig nach außen gerichtet sind, wobei die drei Achsen der trilobalen Form winkelig voneinander beabstandet sind und der Kern eine dreieckige Symmetrie aufweist, wobei der Mantel 10-90 Gew.-% der Faser aufweist und der Kern 90-10 Gew.-% der Faser aufweist, und wobei der Mantel aus einem Co-Polyester, einem Polyolefin oder einem Olefin-Copolymer hergestellt ist und ein für den Kern verwendetes Polymer ein Polyolefin oder ein Olefin-Copolymer oder ein Polyester oder ein Co-Polyester ist, und der Kern einen trilobalen oder deltaförmigen Querschnitt aufweist, der mit dem äußeren trilobalen Querschnitt übereinstimmt, und der Mantel eine formangeglichene Schicht auf dem Kern ist.

2. Bikomponenten-Stapelfaser oder -Kurzschnittfaser nach Anspruch 1, wobei der Mantel aus Polyethylen (PE) hergestellt ist und der Kern aus Polypropylen (PP) hergestellt ist.

3. Bikomponenten-Stapelfaser oder -Kurzschnittfaser nach Anspruch 1 oder 2, wobei der Mantel aus Polyethylen hergestellt ist und zwischen 30-70 Gew.-% der Faser aufweist und der Kern aus Polypropylen hergestellt ist und zwischen 70-30 Gew.-% der Faser aufweist.

4. Bikomponenten-Stapelfaser oder -Kurzschnittfaser nach einem der vorhergehenden Ansprüche, bei der der Endtiter zwischen 0,5 und 35 dtex, vorzugsweise zwischen 0,9 und 17 dtex liegt.

5. Vliesstoffstruktur mit einer Verflechtung von Stapelfasern oder Kurzschnittfasern, wobei die Stapelfasern oder Kurzschnittfasern Bikomponentenfasern umfassen, die thermisch an Bikomponentenfasern gebunden sind, wobei die Bikomponentenfasern einem der vorhergehenden Ansprüche entsprechen.

6. Vliesstruktur nach Anspruch 5, wobei der Mantel aus Polyethylen (PE) hergestellt ist und der Kern aus Polypropylen (PP) hergestellt ist.

7. Vliesstoffstruktur nach einem der Ansprüche 5 oder 6, wobei die Vliesstoffstruktur ein Grundgewicht zwischen 12 g/m² und 170 g/m² aufweist.

8. Vliesstoffstruktur, umfassend trilobal geformte Bikomponenten-Stapelfasern oder -Kurzschnittfasern nach einem der Ansprüche 1 bis 4, wobei die Fasern mit sich selbst oder mit anderen Bikomponentenfasern und/oder mit Monokomponentenfasern verbunden sind.

9. Verwendung der Vliesstoffstruktur nach einem der Ansprüche 5 bis 8 als Oberflächenlage in absorbierenden Gegenständen oder Verwendung in einem absorbierenden Gegenstand, der eine flüssigkeitsdurchlässige, dem Körper zugewandte Deckschicht, eine flüssigkeitsundurchlässige, der Kleidung zugewandte Sperrschicht und einen absorbierenden Kern zwischen der Deckschicht und der Sperrschicht umfasst, wobei die Deckschicht die Vliesstoffstruktur nach einem der Ansprüche 5 bis 8 umfasst.

10. Verwendung nach Anspruch 9 in absorbierenden Gegenständen, die unter der Oberflächenschicht eine Aufnahme-/Transportschicht und eine absorbierende Schicht sowie eine flüssigkeitsundurchlässige Trägerschicht enthalten.

11. Verwendung der Vliesstoffstruktur nach einem der Ansprüche 5 bis 8 in einem Filter in einem Hygieneprodukt, in einem Teppich, einem Vorleger oder einer Matte, einem Polster oder in einem Trocken- oder Feuchttuch.

## Revendications

1. Fibre ultracourte ou discontinue à deux composants comprenant une âme et une gaine, la gaine et l'âme ayant des points de fusion différents, le point de fusion de la gaine étant inférieur au point de fusion de l'âme, la fibre à deux composants ayant une section transversale extérieure de forme trilobée, dans laquelle la fibre a une symétrie triangulaire, chaque lobe de la matière de gaine ayant des axes rayonnant à partir de l'âme, les trois axes de la forme trilobée étant espacés de manière angulaire les uns par rapport aux autres, et dans laquelle l'âme a une symétrie triangulaire, la gaine faisant entre 10 et 90 % en poids de la fibre et l'âme faisant entre 90 et 10 % en poids de la fibre et dans laquelle la gaine est faite d'un copolyester, d'une polyoléfine, ou d'un copolymère d'oléfine, et un polymère utilisé pour l'âme est une polyoléfine, ou un copolymère d'oléfine, ou un polyester ou un copolyester, et l'âme a une section transversale en forme de delta ou à trois lobes conforme à la section transversale extérieure de forme trilobée, et la gaine est une couche de faible épaisseur sur l'âme.

2. Fibre ultracourte ou discontinue à deux composants selon la revendication 1, dans laquelle la gaine est faite de polyéthylène (PE) et l'âme est faite de polypropylène (PP).

3. Fibre ultracourte ou discontinue à deux composants selon la revendication 1 ou la revendication 2, dans laquelle la gaine est faite de polyéthylène et fait entre 30 et 70 % en poids de la fibre et dans laquelle l'âme est faite de polypropylène et fait entre 70 et 30 % en poids de la fibre.

4. Fibre ultracourte ou discontinue à deux composants selon l'une quelconque des revendications précédentes, dans laquelle le titre final est entre 0,5 et 35 dtex, de préférence entre 0,9 et 17 dtex.

5. Structure non tissée ayant un enchevêtrement de fibres ultracourtes ou discontinues, les fibres ultracourtes ou discontinues comprenant des fibres à deux composants liées de façon thermique à des fibres à deux composants, les fibres à deux composants étant selon l'une quelconque des revendications précédentes.

6. Structure de non-tissé selon la revendication 5, dans laquelle la gaine est faite de polyéthylène (PE) et l'âme est faite de polypropylène (PP).

7. Structure non tissée selon l'une quelconque des revendications 5 ou 6, dans laquelle la structure non tissée a un poids de base entre 12 gsm et 170 gsm.

8. Structure non tissée comprenant des fibres de forme trilobée ultracourtes ou discontinues à deux composants selon l'une quelconque des revendications 1 à 4, lesquelles fibres sont liées entre elles ou à d'autres fibres à deux composants et/ou à des fibres à un seul composant.

9. Utilisation de la structure non tissée selon l'une quelconque des revendications 5 à 8 en tant que feuille superficielle dans des articles absorbants ou utilisation dans un article absorbant comprenant une couche de couverture perméable au liquide côté corps, une couche de barrière imperméable au liquide côté vêtement et une âme absorbante entre la couche de couverture et la couche de barrière, la couche de couverture comprenant la structure non tissée selon l'une quelconque des revendications 5 à 8.

10. Utilisation selon la revendication 9, dans des articles absorbants qui incluent au-dessous de la feuille superficielle une feuille d'acquisition/transport et une feuille absorbante et une feuille de support imperméable au liquide.

11. Utilisation de la structure non tissée selon l'une quelconque des revendications 5 à 8, dans un filtre dans un produit d'hygiène, dans un tapis, une carpette ou une natte, un tissu d'ameublement, ou dans une lingette sèche ou humide.
